# EUROPEAN PATENT SPECIFICATION

(11) **EP 2 886 138 B1**
(45) Date of publication and mention of the grant of the patent: **19.07.2017**
(21) Application number: 13829908.6
(22) Date of filing: 12.08.2013
(51) Int. Cl.: A61M 1/06, A61F 7/08, A41C 3/00, A61F 7/00

(54) **BREAST PUMP WITH ELECTRIC HEATING COVER**
BRUSTPUMPE MIT ELEKTRISCHER ERWÄRMUNGSABDECKUNG
TIRE-LAIT À COUVERCLE CHAUFFANT ÉLECTRIQUE

(30) Priority: 15.08.2012 CN 201210290386
(43) Date of publication of application: 24.06.2015
(73) Proprietor: Lactans Healthcare Inc., Ningbo 315016 (CN)
(72) Inventor: HU, Min, Ningbo, Zhejiang 315012 (CN)
(74) Representative: Hryszkiewicz, Danuta
(86) International application number: PCT/CN2013/000947
(87) International publication number: WO 2014/026466

(56) References cited:
- EP-A1- 1 894 548
- CN-A- 1 913 930
- CN-A- 102 805 884
- CN-U- 201 840 575
- CN-U- 202 154 891
- CN-U- 202 844 195
- CN-U- 202 844 195
- CN-Y- 2 237 428
- CN-Y- 2 242 701
- CN-Y- 2 643 920
- TW-U- M 296 048
- US-A1- 2006 247 559
- US-A1- 2006 264 816
- US-A1- 2008 262 419
- US-A1- 2008 312 586

## Description

The invention relates to a breast pump, and more particularly to a breast pump with an electric heating cup.

Breast pump is one of the essential tools for almost all lactating women. During the lactation period, the mother may have to take medicines due to diseases and therefore the lactation must be stopped temporarily in order to avoid the influence of the medicines on the baby. The normal breastfeeding may also be affected by outing or working of the mother. In addition, for some other reasons, such as the lactating method or postpartum depression, circumstances like milk siltation or blockage of mammary duct caused by non-smooth milk transportation may occur during the normal lactation period of the mother. However, the milk secretion will not be stopped in the above special circumstances, and therefore the breast pump is required to suck the milk out of the breast, otherwise, the milk siltation in the mammary duct and the blockage of the mammary duct may occur, which further leads to breast masses and makes the mother feel breast swelling and pain. When the milk silts the mammary duct for certain hours or one day, acute mastitis is often resulted, or in more serious circumstance secondary breast abscess may be resulted. All such illnesses bring great suffers to the young mother as well as seriously affect postpartum recovery of the mother and damage maternal and child health.

The breast pump for extracting milk is very common and often adopts a vacuum pump device to connect with one or two brassieres and a collection container, such as a milk bottle for collecting the extracted milk. The vacuum pump enables the brassieres disposed on the breasts to produce a negative pressure and controls the negative pressure to extract the milk and collect the milk in the milk bottle.

Far infrared heating therapy within a safe temperature range of the body has significant physical therapy effect. Since the vibration frequency of the far infrared ray is basically coincident with that of molecules within human cells, when the far infrared rays act on the skin, a large amount of the energy is absorbed by the skin. The absorbed energy is then converted into heat energy, so that the skin temperature increases, which stimulates a thermoreceptor within the skin and further initiates thalamus reflection, thereby leading to relaxation of vascular smooth muscle, angiectasis, and accelerated blood circulation. In another aspect, under the thermal effects, vasoactive substances are released, the vascular tension is decreased, superficial small arteries, superficial capillaries, and superficial vein dilate, and the blood circulation accelerates, thereby improving the blood circulation. Tests from Institute of Hematology, Chinese Academy of Medical Sciences indicates that 20 min of far infrared heating therapy is able to increase the blood flow of the microcirculation by 114%. Medical far infrared ray, also called long-wave infrared ray, having a wavelength of between 4 and 400 µm, is able to penetrate human tissue to a depth of between 3 and 5 cm.

The mammary duct has a physical structure similar to the blood vessel and is formed by smooth muscles. Besides, milk collecting ducts are arranged in the mastoid sinus beneath the mammary areola, and the milk secreted by the mammary gland tissue is accumulated in the mastoid sinus. Due to some reasons, such as insufficient suction of the baby, depression of the mother, poor health of the mother, and improper or overuse of the conventional breast pump, the milk collecting ducts are prone to adhere to one another and blockage thereof occur, thereby resulting in difficulties in milk secretion or breast swelling. Thus, when the far infrared rays act on the mammary areola, the smooth muscles of the mammary duct (particularly the milk collecting ducts) relax, the mammary duct dilates, the blockage of the mammary duct is quickly alleviated, the mammary areola is softened, and the milk flow is accelerated, so that the milk is easily sucked out by the baby or by the breast pump. As the milk flow is accelerated, the thalamus reflection is initiated and more prolactin is secreted, so that the amount of the secreted milk is increased. The latest international medical research shows that insufficient breastfeeding and too short the lactation period have a certain direct relation with the breast cancer of women. According to 5 years of research of 100 top level scientists from 35 countries, the latest cancer research report is disclosed to the world by the World Cancer Research Fund and the American Institute for Cancer Research on October 31, 2007. It is confirmed for the first time by authority in the world that six months of exclusive breastfeeding is capable of effectively preventing the mother from breast cancer as well as preventing the child from overweight and obesity.

The mother should avoid directly contact with cold or cool objects in a certain primary period after the childbirth. When the conventional breast pump is used in winter, the breast pump in the absence of the heating function is directly contact with the skin of the breast, the mammary duct is contracted because of the low temperature of the breast pump, thereby affecting the milk secretion or even resulting in non-smooth milk transportation. Some mothers may firstly immersing the breast pump in hot water before the use, however, such method is time consuming and labor consuming, and the temperature quickly decreases.

Thus, the far infrared heating therapy is conducted on the mammary areolae of the lactating mother to ensure that with the help of the breast pump, the milk remaining or silting in the breast is evacuated timely and the blockage of mammary duct is prevented or evacuated timely, which is of great significance for improving the quality of the breastfeeding, promoting the exclusive breastfeeding rate of the human being, and ensuring the maternal and child health.

Chinese Application No. 200680047746.1 (Publication No. CN101340937A) discloses a breast pump employing carbon fiber, which is disposed outside the bell mouth thereof, as a heating element. The temperature of the heating element is controlled by an external temperature sensor. Thus, the breast pump has a complex structure and manufacturing process. In addition, the heating element is subject to the natural properties of the carbon fiber, which often has disadvantages such as nonuniform hot spots, irregular resistance variation, and the occurrence of focal temperature. Furthermore, conventional breast pumps are difficult to clean and sterilize, so that the breast milk mist tends to precipitate and accumulate in the pipes, casing the generation of mildew and bacteria.

In addition, CN202154891 U discloses a breast pump, the breast pump including an electric heating unit, and the electric heating unit including an upper layer, a contact layer, a vibration device, a control unit, a vibration, and a variable resistance electric heating element. The upper layer and the contact layer are made of silica gel and textile material. In addition, the variable resistance electric heating element generates heat, the control unit controls the heating temperature, and the vibration device generates vibration.

In addition, EP1894548 A1 discloses a heating pad which includes a heating storage medium, a resistance electric heating element, and a thermally insulating layer. When in use, the resistance electric heating element is energized, the resistance electric heating element generates heat, the heat transfers to heat the heating storage medium, and the thermally insulating layer is used to decrease the heat loss.

In addition, US20080312586 A1 discloses a breast shield which includes an electrical resistance heating element, and the electrical resistance heating element is made of a plastic material and an electrically conductive additive. When in use, the electrical resistance heating element is energized and generates heat.

It is one objective of the invention to provide a breast pump that has constant and safe heat supply. The invention is defined by the appended claims.

To achieve the above objective, in accordance with one embodiment of the invention, there is provided a breast pump comprising a container; a host unit disposed above and communicates with the container; and an electric heating cup disposed at a front end of the host unit and communicates with the container; the host unit comprising a miniature electric vacuum pump. The electric heating cup comprises an inner cover and an outer cover which are integrated; an electric heating element and heat insulation foam are disposed between the inner cover and the outer cover. The heat insulation foam is disposed between the electric heating element and the outer cover. Positive and negative electrodes of the electric heating element are connected to conducting plates disposed in an inner side of the outer cover via connection wires. The conducting plates extend to an outer surface of the outer cover and are in electric connection to contact chips disposed at a lower front end of the host unit.

A connecting piece is disposed above the container, and a lower end of the connecting piece is in seal connection to the container; the connecting piece comprises a conduit at a front end thereof and is in fixed seal connection to the electric heating cup through the conduit; the connecting piece comprises a through hole, and the host unit comprises a suction pipe and is fixed on the connecting piece by inserting the suction pipe into the through hole; and the suction pipe is inserted into the through hole so that the host unit, the connecting piece, and the container are tightly connected.

According to the invention the electric heating element is an electric heating film, which is fixed on one side of the inner cover facing the outer cover; the heat insulation foam is fixedly attached to one side of the electric heating film facing the outer cover; one end of the conducting plates is disposed between the inner cover and the outer cover, and is connected to the electric heating film via connection wires; and the other end of the conducting plates is exposed out of the outer cover.

The electric heating film comprises a plurality of sheets affixed at intervals on the side of the inner cover facing the outer cover, the sheets are connected in parallel with one another and all connected to the conducting plates; or the electric heating film is an integrated ring, positive and negative electrodes thereof are connected to the conducting plates.

The inner cover and the outer cover are both made of hard plastic material free of bisphenol amine.

The electric heating film is made of polytetrafluoroethylene-based temperature-constant far infrared electric heating material.

The conducting plates and the contact chips are gold-plated copper sheets.

Advantages of the invention are summarized as follows. The breast pump comprises the electric heating cup comprising the inner cover and the outer cover, and the electric heating element clamped between the inner cover and the outer cover, all of which are fabricated into an integrated structure by secondary injection molding, ultrasonic welding technology, and lathe grinding and polishing processing. The electric heating element is fixed between the inner cover and the outer cover, so it is not easy to detach, convenient for cleaning, and has impact and beautiful appearance. The conducting plates of the electric heating cup are fixed on the outer cover through secondary injection molding and are in electric connection to the contact chips at the lower front end of the host unit. Thus, the structure is simple, compact, stable and convenient for use and mass production. The resulting breast pump is made of opaque material, so the users cannot see the electric heating element and connection wires, providing the users with the sense of safety.
FIG. 1 is a stereogram of a breast pump in accordance with one embodiment of the invention;
FIG. 2 is a local sectional view of a breast pump in accordance with one embodiment of the invention;
FIG. 3 is a local exploded view of a breast pump in accordance with one embodiment of the invention;
FIG. 4 is a stereogram of an electric heating cup of a breast pump in accordance with one embodiment of the invention;
FIG. 5 is a stereogram of a host unit of a breast pump in accordance with one embodiment of the invention.

For further illustrating the invention, experiments detailing a breast pump are described below. It should be noted that the following examples are intended to describe and not to limit the invention.

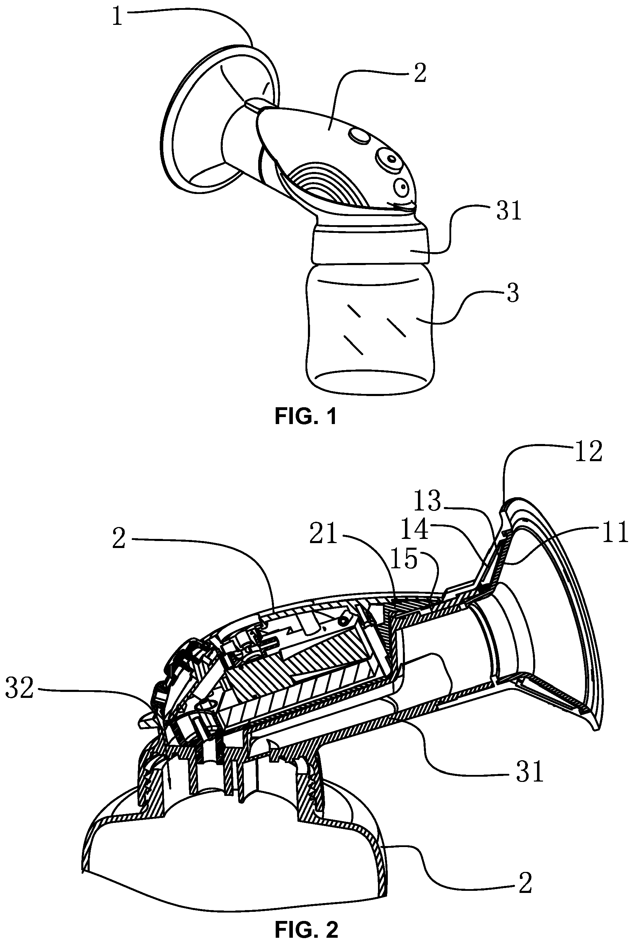

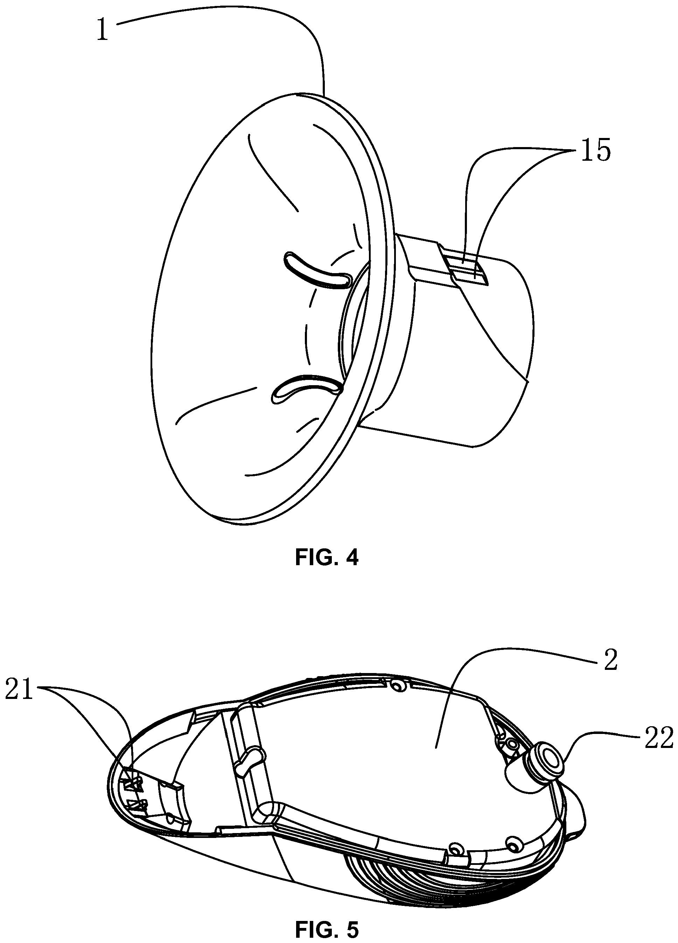

As shown in FIGS. 1-5 a breast pump of the invention comprises a container 3, a host unit 2 disposed above and communicating with the container, an electric heating cup 1 disposed at the front end of the host unit 2 and communicating with the container 3.

The host unit 2 comprises a miniature electric vacuum pump, a magnetic valve configured to generate vacuum negative pressure, an air loop comprising an air inlet and an air outlet, an integrated circuit board, and a rechargeable lithium battery for supply power for the breast pump. The front end of the electric heating cup 1 is in the shape of a horn, and its diameter is slightly larger than that of female mammary areola, so that the electric heating cup 1 better contacts the female mammary areola. The rear end of the electric heating cup 1 has an inner diameter larger than that of mammary papilla, which, preferably, is twice the outer diameter of the mammary papilla in lactation period. The rear end of the electric heating cup 1 is a straight pipe with a round cross section and has certain length thereby ensuring enough space for the mammary papilla and protecting the mammary papilla from squeezing.

Preferably, the breast pump comprises a connecting piece 3 at the top thereof. The connecting piece 31 is disposed above the container 3 and the lower end thereof is in seal connection to the container 3 by thread screw. A silicone flat washer 34 is disposed between the connecting piece and the container 3. A conduit protrudes from a front end of the connecting piece 31 and is in fixed seal connection to the rear end of the electric heating cup 1. The opening of the conduit is a straight pipe with a round cross section, on which an O-shaped silicone seal ring 33 is disposed. The connecting piece 31 comprises a through hole 32. The host unit 2 is fixed on the connecting piece 31 and comprises a suction pipe 22; the suction pipe 22 is inserted into the through hole 32 and the connecting piece 31 is in seal connection to the container 3. The through hole 32 is also provided with an O-shaped seal ring 321. The front end of the connecting piece 31 is connected to the rear end of the electric heating cup 1 via plugging, screw thread, or bayonet screwing.

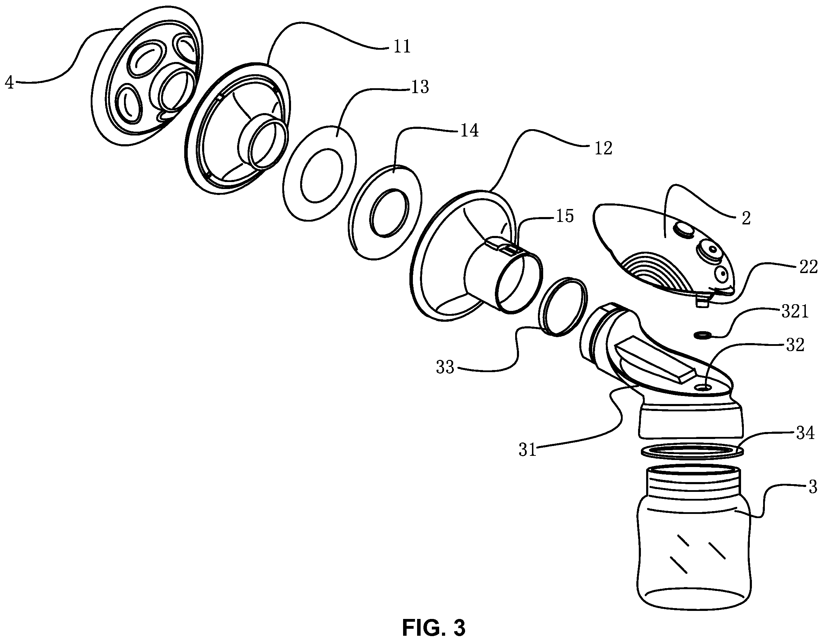

The electric heating cup 1 comprises an inner cover 11 and an outer cover 12, as shown in FIG. 3. The electric heating element and the heat insulation foam 14 are disposed between the inner cover 11 and the outer cover 12. The electric heating element is an electric heating film, electric heating wire, or other electric heating materials. The electric heating element is an electric heating film 13, which is fixed on one side of the inner cover 11 facing the outer cover 12; the heat insulation foam 14 is fixedly attached to one side of the electric heating film 13 facing the outer cover 12. The heat insulation foam 14 is disposed between the electric heating element and the outer cover 12; positive and negative electrodes of the electric heating element are connected to conducting plates 15 disposed in an inner side of the outer cover 12 via connection wires, respectively; the conducting plates 15 extend to an outer surface of the outer cover 12 and are in electric connection to contact chips 21 disposed at a lower front end of the host unit 2. One end of the conducting plates 15 is disposed between the inner cover 11 and the outer cover 12, and is connected to the electric heating film 13 via connection wires; and the other end of the conducting plates 15 is exposed out of the outer cover 12.

When the electric heating cup 1 is connected to the front end of the connecting piece 31, the host unit 2 is clasped on the connecting piece 31, so that the contact chips 21 disposed at the lower front end of the host unit 2 contact the conducting plates 15. Thus, the power source of the host unit supplies power for the electric heating cup 1 via the contact chips 21 and the conducting plates 15.

The inner cover 11 and the outer cover 12 are both made of food grade hard plastic material free of bisphenol amine, for example, polypropylene (PP), preferably, TRITAN copolymer from Eastman Chemical Company of U. S. A.

Upon manufacturing the electric heating cup 1, the outer cover 12 is prepared using the secondary injection molding technology. First, two conducting plates 15 are molded by injection together to form an insert, which is further molded by injection with the outer cover 12, so that the conducting plates 15 and the outer cover 12 are integrated. Specifically, one end of the conducting plates 15 is disposed at the inner side of the outer cover 12, and the other end of the conducting plates 15 is exposed out of the outer cover 12. The exposed part of the conducting plates 15 is in seamless connection to the outer surface of the outer cover 12, and both are in the same plane without height difference. Thereafter, the electric heating film 13 and the heat insulation foam 14 are fixed on one side of the inner cover 11 facing the outer cover 12, and the positive and negative electrodes of the electric heating film 13 are connected to the conducting plates, respectively. In the presence of ultrasonic wave, the inner cover 11 and the outer cover 12 of the electric heating cup 1 are welded and sealed to form an integrated structure. Look from the outside surface, no connection wires and socket connectors can be observed. Only two conducting plates 15 disposed in parallel on the outer side of the outer cover 12 can be observed. That the contact chips 21 disposed at the lower front end of the host unit 2 are connected to the conducting plates 15 on the outer surface of the outer cover 12 by contact makes the breast bump have a simple structure, convenient power supply and beautiful appearance.

The electric heating film 13 is made of polytetrafluoroethylene-based temperature-constant far infrared electric heating material, has a working voltage of 7.4 V and a thickness of 0.2-0.5 mm. The electric heating film 13 can be, as shown in FIG. 3, an integrated ring circling the protrusion of the inner cover 11, or optionally, comprises a plurality of sheets affixed at intervals on the side of the inner cover 11 facing the outer cover 12. Preferably, the electric heating film 13 employs 3-5 sheets having a size of 15 mm x 30 mm. The sheets are connected in parallel with one another via conductive copper foils. The conductive copper foils are fixed on the electric heating film through conductive adhesives, preferably. The electric heating film 13 can be fixed on the inner cover 13 through conventional double-sided foam tape. One end of the connection wire is welded on the conductive copper foils of the electric heating film by solder tin. If the electric heating film comprises a plurality of sheets, the sheets are connected in parallel with one another and connected to the conducting plates 15. The heat insulation foam 14 is, preferably, flexible cotton sheet with back adhesive and having a thickness of 3-5 mm, which can be firmly attached to the electric heating film 13, thereby preventing the detaching of the electric heating film from the inner cover, protecting the far infrared energy from emitting outside to the outer cover 12, so that the far infrared energy is concentrated to irradiate to the areola mammae thereby ensuring good health care effect and saving electric energy.

The working voltage of the electric heating film 13 is 7.4 V, the resistance thereof is about 40 ohm, and the power thereof is less than 1.4 W. Such low power enables the host unit to employ a miniature rechargeable lithium battery, so that the breast pump is small and convenient for carrying. In addition, the working temperature of the electric heating cup is constantly at 38-40°C, which is the optimal temperature for the hot care of the areola mammae, so no matter in winter or summer, the use of the breast pump makes users feel comfortable.

Optionally, a detachable silicone protection pad 4 is disposed at the inner side of the electric heating cup 1. The silicone protection pad 4 has adapted curve surface with the electric heating cup and comprises convex points for massage, thereby improving the usage comfort.

Preferably, the conducting plates and the contact chips are a gold-plated copper sheet. The gold-plated copper sheet has excellent electrical conductivity, and looks shining, easy to clean and difficult to rust, which ensures the beautiful appearance of the product, and the gold-plated copper sheet is more suitable for preparation of children's containers meeting the international standard of environmental protection.

The breast pump comprises the electric heating cup comprising the inner cover and the outer cover, and the electric heating element clamped between the inner cover and the outer cover, all of which are fabricated into an integrated structure by secondary injection molding and ultrasonic welding technology. The electric heating element is fixed between the inner cover and the outer cover, so it is not easy to detach, convenient for cleaning, and has impact and beautiful appearance.

The conducting plates are fixed on the outer cover through secondary injection molding and are in electric connection to the contact chips at the lower front end of the host unit. Thus, the structure is simple, compact, stable and convenient for use and mass production. The resulting breast pump is made of opaque material, so the users cannot see the electric heating element and connection wires, providing the users with the sense of safety.

## Claims

1. A breast pump with an electric heating cup, comprising: a container (3); a host unit (2) disposed above and communicates with the container (3); and an electric heating cup (1) disposed at a front end of the host unit (2) and communicates with the container (3); the host unit (2) comprising a miniature electric vacuum pump; wherein the electric heating cup (1) comprises an inner cover (11) and an outer cover (12) which are integrated;
an electric heating film (13) and heat insulation foam (14) are disposed between the inner cover (11) and the outer cover (12);
each of the inner cover (11) and the outer cover (12) is made of a hard plastic material free of bisphenol amine;
the electric heating film (13) is made of a polytetrafluoroethylene-based temperature-constant far infrared electric heating material, wherein when the polytetrafluoroethylene-based temperature-constant far infrared electric heating material is energized, the polytetrafluoroethylene-based temperature-constant far infrared electric heating material produces far infrared rays and has a substantially constant temperature;
the heat insulation foam (14) is disposed between the electric heating film (13) and the outer cover (12);
positive and negative electrodes of the electric heating film (13) are connected to electrically conducting plates (15) disposed in an inner side of the outer cover (12) via connection wires; and
the electrically conducting plates (15) extend to an outer surface of the outer cover (12) and are in electric connection to contact chips (21) disposed at a lower front end of the host unit (2).

2. The breast pump of claim 1, **characterized in that** a connecting piece (31) is disposed above the container (3), and a lower end of the connecting piece (31) is in seal connection to the container (3);
the connecting piece (31) comprises a conduit at a front end thereof and is in fixed seal connection to the electric heating cup (1) through the conduit;
the connecting piece (31) comprises a through hole (32), and the host unit (2) comprises a suction pipe (22) and is fixed on the connecting piece (31) by inserting the suction pipe (22) into the through hole (32); and
the suction pipe (22) is inserted into the through hole (32) so that the host unit (2), the connecting piece (31), and the container (3) are tightly connected.

3. The breast pump of claim 1, **characterized in that** the electric heating film (13) is fixed on one side of the inner cover (11) facing the outer cover (12); the heat insulation foam (14) is fixedly attached to one side of the electric heating film (13) facing the outer cover (12); one end of the electrically conducting plates (15) is disposed between the inner cover (11) and the outer cover (12), and is connected to the electric heating film (13) via connection wires; and the other end of the electrically conducting plates (15) is exposed out of the outer cover (12).

4. The breast pump of claim 3, **characterized in that** the electric heating film (13) comprises a plurality of sheets affixed at intervals on the side of the inner cover (11) facing the outer cover (12), the sheets are connected in parallel with one another and all connected to the electrically conducting plates (15); or the electric heating film (13) is an integrated ring, positive and negative electrodes thereof are connected to the electrically conducting plates (15).

5. The breast pump of any one of claims 1-4, **characterized in that** the electrically conducting plates (15) and the contact chips (21) are gold-plated copper sheets.

## Patentansprüche

1. Milchpumpe mit einem elektrischen Heizbecher, umfassend: einen Behälter (3); eine Host-Einheit (2), die über dem Behälter (3) angeordnet ist und mit diesem kommuniziert; und einen elektrischen Heizbecher (1), der an der Vorderseite der Host-Einheit (2) angeordnet ist und mit dem Behälter (3) kommuniziert; wobei die Host-Einheit (2) eine Miniatur-Vakuumpumpe umfasst; wobei
der elektrische Heizbecher (1) eine innere Abdeckung (11) und eine äußere Abdeckung (12) umfasst, die eingebaut sind;
eine elektrische Heizfolie (13) und ein Wärmeisolierungsschaum (14) zwischen der inneren Abdeckung (11) und der äußeren Abdeckung (12) angeordnet sind;
die innere Abdeckung (11) und die äußere Abdeckung (12) jeweils aus einem harten Kunststoffmaterial herstellt sind, das frei von Bisphenol-Amin ist;
die elektrische Heizfolie (13) aus einem auf Polytetrafluorethylen basierten elektrischen Ferninfrarot-Heizmaterial mit konstanter Temperatur besteht, wobei das auf Polytetrafluorethylen basierte elektrische Ferninfrarot-Heizmaterial mit konstanter Temperatur unter Spannung steht, das auf Polytetrafluorethylen basierte elektrische Ferninfrarot-Heizmaterial mit konstanter Temperatur Ferninfrarotstrahlen produziert und eine im Wesentlichen konstante Temperatur aufweist;
der Wärmeisolierungsschaum (14) zwischen der elektrischen Heizfolie (13) und der äußeren Abdeckung (12) angeordnet ist;
positive und negative Elektroden der elektrischen Heizfolie (13) mit elektrisch leitfähigen Platten (15), die in einer Innenseite der äußeren Abdeckung (12) angeordnet sind, durch Anschlussdrähte verbunden sind; und
die elektrisch leitfähigen Platten (15) sich zu einer Außenfläche der äußeren Abdeckung (12) erstrecken und in elektrischer Verbindung mit Kontaktchips (21) stehen, die an einem unteren vorderen Ende der Host-Einheit (2) angeordnet sind.

2. Milchpumpe nach Anspruch 1, **dadurch gekennzeichnet, dass** ein Verbindungsstück (31) über dem Behälter (3) angeordnet ist, und ein unteres Ende des Verbindungsstücks (31) in einer dichtenden Verbindung mit dem Behälter (3) steht;
das Verbindungsstück (31) umfasst eine Leitung an einem vorderen Ende davon und steht in einer nicht lösbaren dichtenden Verbindung mit dem elektrischen Heizbecher (1) durch die Leitung;
das Verbindungsstück (31) umfasst eine Durchgangsbohrung (32), und die Host-Einheit (2) umfasst ein Ansaugrohr (22) und wird an dem Verbindungsstück (31) befestigt, indem das Ansaugrohr (22) in die Durchgangsbohrung (32) eingeführt wird; und
das Ansaugrohr (22) wird in die Durchgangsbohrung (32) eingeführt, so dass die Host-Einheit (2), das Verbindungsstück (31) und der Behälter (3) fest miteinander verbunden sind.

3. Milchpumpe nach Anspruch 1, **dadurch gekennzeichnet, dass** die elektrische Heizfolie (13) auf einer Seite der Innenabdeckung (11) befestigt ist, die der äußeren Abdeckung (12) gegenüberliegt; der Wärmeisolierungsschaum (14) ist fest auf der einen Seite der elektrischen Heizfolie (13) angebracht, die der äußeren Abdeckung (12) gegenüberliegt; ein Ende der elektrisch leitfähigen Platten (15) ist zwischen der inneren Abdeckung (11) und der äußeren Abdeckung (12) angeordnet und ist mit der elektrischen Heizfolie (13) über Anschlussdrähte verbunden; und das andere Ende der elektrisch leitfähigen Platten (15) wird unter der äußeren Abdeckung (12) freigelegt.

4. Milchpumpe nach Anspruch 3, **dadurch gekennzeichnet, dass** die elektrische Heizfolie (13) eine Vielzahl von Bahnen umfasst, die in Abständen auf der Seite der inneren Abdeckung (11) befestigt sind, die der äußeren Abdeckung (12) gegenüberliegt, wobei die Bahnen parallel miteinander verbunden sind und alle mit den elektrisch leitfähigen Platten (15) verbunden sind; oder die elektrische Heizfolie (13) ist ein eingebauter Ring, dessen positive und negative Elektroden mit den elektrisch leitfähigen Platten (15) verbunden sind.

5. Milchpumpe nach einem der Ansprüche 1-4, **dadurch gekennzeichnet, dass** die elektrisch leitfähigen Platten (15) und die Kontaktchips (21) vergoldete Kupferplatten sind.

## Revendications

1. Pompe tire-lait avec une coupelle de chauffage électrique, comprenant : un récipient (3) ; une unité hôte (2) disposée au-dessus et qui communique avec le récipient (3) ; et une coupelle de chauffage électrique (1) disposée à l'extrémité avant de l'unité hôte (2) et qui communique avec le récipient (3) ; l'unité hôte (2) comprenant une pompe à vide électrique miniature ; dans laquelle
la coupelle de chauffage électrique (1) comprend une protection intérieure (11) et une protection extérieure (12) qui sont intégrées ;
un film de chauffage électrique (13) et une mousse isolante thermique (14) sont disposés entre la protection intérieure (11) et la protection extérieure (12) ;
chacune de la protection intérieure (11) et de la protection extérieure (12) est en matière plastique dure exempte de bisphénol amine ;
le film de chauffage électrique (13) est en matériau de chauffage électrique à infrarouges lointains à température constante à base de polytétrafluoroéthylène, dans lequel, lorsque le matériau de chauffage électrique à infrarouges lointains à température constante à base de polytétrafluoroéthylène est excité, le matériau de chauffage électrique à infrarouges lointains à température constante à base de polytétrafluoroéthylène produit des rayons infrarouges lointains et a une température sensiblement constante ;
la mousse isolante thermique (14) est disposée entre le film de chauffage électrique (13) et la protection extérieure (12) ;
les électrodes positives et négatives du film de chauffage électrique (13) sont connectées à des plaques électriquement conductrices (15) disposées sur un côté intérieur de la protection extérieure (12) par l'intermédiaire de fils de connexion ; et
les plaques électriquement conductrices (15) se prolongent vers une surface extérieure de la protection extérieure (12) et sont en connexion électrique avec des puces de contact (21) disposées sur une extrémité avant inférieure de l'unité hôte (2).

2. Pompe tire-lait selon la revendication 1, **caractérisée en ce qu'**une pièce de connexion (31) est disposée au-dessus du récipient (3) et une extrémité inférieure de la pièce de connexion (31) est en connexion étanche avec le récipient (3) ;
la pièce de connexion (31) comprend une conduite en son extrémité avant et est en connexion étanche fixe avec la coupelle de chauffage électrique (1) par le biais de la conduite ;
la pièce de connexion (31) comprend un trou traversant (32), et l'unité hôte (2) comprend un tuyau d'aspiration (22) et est fixée sur la pièce de connexion (31) en insérant le tuyau d'aspiration (22) dans le trou traversant (32) ; et
le tuyau d'aspiration (22) est inséré dans le trou traversant (32) de sorte que l'unité hôte (2), la pièce de connexion (31) et le récipient (3) soient étroitement connectés.

3. Pompe tire-lait selon la revendication 1, **caractérisée en ce que** le film de chauffage électrique (13) est fixé sur un côté de la protection intérieure (11) en regard de la protection extérieure (12) ; la mousse isolante thermique (14) est attachée de manière fixe à un côté du film chauffant électrique (13) en regard de la protection extérieure (12) ; une extrémité des plaques électriquement conductrices (15) est disposée entre la protection intérieure (11) et la protection extérieure (12) et est connectée au film de chauffage électrique (13) par l'intermédiaire de fils de connexion ; et l'autre extrémité des plaques électriquement conductrices (15) est exposée hors de la protection extérieure (12).

4. Pompe tire-lait selon la revendication 3, **caractérisée en ce que** le film de chauffage électrique (13) comprend une pluralité de feuilles fixées à intervalles sur le côté de la protection intérieure (11) en regard de la protection extérieure (12), les feuilles sont connectées parallèlement les unes par rapport aux autres et sont toutes connectées aux plaques électriquement conductrices (15) ; ou le film de chauffage électrique (13) est une bague intégrée dont ses électrodes positives et négatives sont connectées aux plaques électriquement conductrices (15).

5. Pompe tire-lait selon l'une quelconque des revendications 1 à 4, **caractérisée en ce que** les plaques électriquement conductrices (15) et les puces de contact (21) sont des feuilles de cuivre plaquées d'or.
